# EUROPEAN PATENT APPLICATION

(11) **EP 1 736 185 A2**
(43) Date of publication of application: **27.12.2006**
(21) Application number: 06076185.5
(22) Date of filing: 07.06.2006
(51) Int. Cl.: A61M 1/36

(54) **Needle disengagement sensing mechanism**

(30) Priority: 20.06.2005 US 156440
(71) Applicant: Delphi Technologies, Inc., Troy, Michigan 48007 (US)
(72) Inventor: Lovejoy, David A., Thiensville, WI 53092 (US); Youngblood, James H., Highland, MI 48357 (US); Voto, Andrew M., Brighton, MI 48116 (US); Sevrain, Christophe, Bloomfield, MI 48304 (US)
(74) Representative: Denton, Michael John

(57) **Abstract**

A needle disengagement sensing mechanism includes a fistula needle that is adapted to carry fluid. An electronic sensing system is operatively connected to the needle. The sensing system is capable of detecting partial or full disengagement of the needle from a position where it is engaged with the fluid. Disengagement detection is a result of i) a comparative analysis of complex impedance measurements of the needle when in and at least partially out of the fluid engaged position and/or ii) a comparative analysis of optical spectroscopic measurements taken through an area of the mechanism adapted to carry the fluid, the measurements being taken when the needle is in and at least partially out of the fluid engaged position. An alarm control conduit operatively connects the sensing system to a hemodialysis machine and/or an electronic device. The alarm control conduit generates an alarm upon detecting at least partial disengagement of the needle.

## Description

### BACKGROUND OF THE INVENTION

The present disclosure relates generally to detecting needle disengagement, and more particularly to mechanisms for sensing needle disengagement.

Hemodialysis treatments use AV fistula needles so that blood may be removed from an arterial side of a patient for dialysis and the cleansed blood returned to the patient via the venous side. In some instances, the needle may become disconnected from the vascular access point of the patient.

One potential problem that may be associated with needle disengagement on the venous side of the vascular access point is that the patient may suffer substantial blood loss. Various hemodialysis machines include visual and/or audio alarms that are generated upon recognition of needle disconnection. The visual and/or audio alarms may be useful to alert another party, for example, a caregiver, of the disengagement, however, if that party is unable to respond, the potential for the patient to lose blood remains.

Various methods are available for detecting disconnection of the needle. One method of detecting needle disengagement involves measuring venous pressure. However, a potential problem with measuring venous pressure is that the device may fail to register a significant pressure change due, at least in part, to "back pressure" forming in the venous blood line. Another method of detecting needle disengagement involves using leaking body fluid to complete circuits and generate an alarm. A potential problem with this method is that the level of the leaking body fluid may be below a threshold level needed to generate the alarm. As such, the leak may go undetected. Still another method of detecting needle disengagement involves measuring the conductivity of the body fluid. A potential problem with this method is that a slight disconnection of the needle may not generate a significant change in the conductivity of the fluid contacting the sensor.

As such, it would be desirable to provide a mechanism that is capable of detecting needle disengagement with a desirable level of sensitivity and/or is capable of shutting down the hemodialysis machine automatically upon recognition of needle disengagement.

### SUMMARY OF THE INVENTION

A needle disengagement sensing mechanism includes a fistula needle that is adapted to carry a fluid. An electronic sensing system is operatively connected to the needle. The electronic sensing system is capable of detecting partial or full disengagement of the fistula needle from a position where it is engaged with the fluid. Disengagement detection is a result of i) a comparative analysis of complex impedance measurements of the needle when in and at least partially out of the fluid engaged position and/or ii) a comparative analysis of optical spectroscopic measurements taken through an area of the mechanism adapted to carry the fluid, the measurements being taken when the needle is in and at least partially out of the fluid engaged position. An alarm control conduit operatively connects the sensing system to a hemodialysis machine and/or an electronic device. The alarm control conduit generates an alarm upon detecting at least partial disengagement of the fistula needle.

### BRIEF DESCRIPTION OF THE DRAWINGS

Objects, features and advantages of embodiments of the present disclosure will become apparent by reference to the following detailed description and drawings, in which like reference numerals correspond to similar, though not necessarily identical components. Reference numerals having a previously described function may not necessarily be described in connection with other drawings in which they appear.
Fig. 1 is a flow diagram depicting embodiments of a method for detecting at least partial disengagement of a fistula needle from a patient;
Fig. 2 is a schematic, partially exploded diagram of an embodiment of a needle disengagement sensing mechanism;
Fig. 3 is a schematic diagram of an embodiment of an electronic sensing system of the needle disengagement sensing mechanism; and
Fig. 4 is a schematic diagram of an alternate embodiment of an electronic sensing system of the needle disengagement sensing mechanism.

### DESCRIPTION OF THE PREFERRED EMBODIMENTS

Embodiment(s) of the device/mechanism and method disclosed herein advantageously measure complex impedance of the needle and/or optical signals of an area of the mechanism/needle, thereby allowing partial or full disengagement to be detected with a desired level of sensitivity. Measuring the complex impedance of the needle and/or optical signals of an area of the mechanism/needle provides substantially increased sensitivity, at least in part, because the measurement is a multivariate measurement. The mechanism has the capability to measure more than one physical, electrical, and/or optical property substantially simultaneously, thereby improving the resolution of the measurement and allowing detection of partial or full needle disengagement with a substantially high degree of confidence. Further, embodiment(s) of the device/mechanism and method disclosed herein advantageously allow the automatic shutdown of a hemodialysis machine upon recognition of partial or full disengagement of a needle from an engaged patient position. The automatic shutdown enables a patient's treatment to be discontinued without the assistance of another person (e.g. a caregiver). Still further, embodiment(s) of the mechanism incorporate measuring complex impedance of the needle and/or optical signals of an area of the mechanism/needle adapted to carry fluid via an electronic sensing system that may be passive or active.

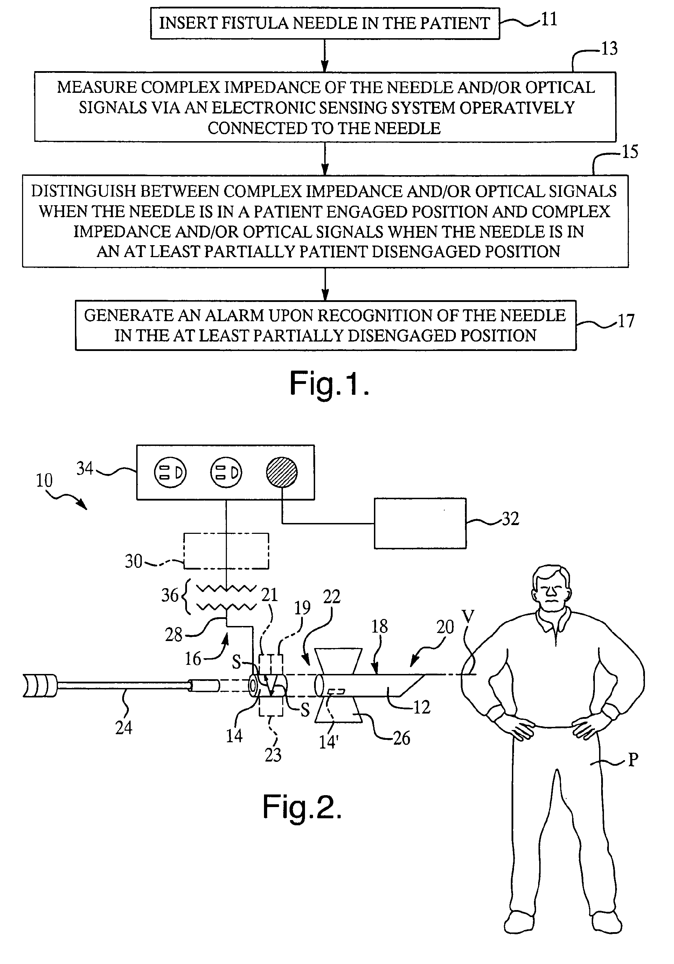

Referring now to Fig. 1, an embodiment of the method for detecting at least partial disengagement of a fistula needle from a patient is depicted. The method includes inserting the needle into the patient, as shown at reference numeral 11. The fistula needle is operatively connected to an electrical sensing system, which in turn is operatively connected to an alarm control conduit. Complex impedance of the needle and/or optical signals is/are measured via the electronic sensing system, as shown at reference numeral 13. The electronic sensing system is capable of distinguishing between complex impedance and/or optical signals when the needle is in a patient engaged position and complex impedance and/or optical signals when the needle is in an at least partially patient disengaged position, as depicted at reference numeral 15. Upon recognition of the needle in the at least partially disengaged position, an alarm is generated by the alarm control conduit, as shown at reference numeral 17. It is to be understood that the method depicted in Fig. 1 will be discussed in further detail hereinbelow in reference to Figs. 2 through 4.

Referring now to Fig. 2, an embodiment of a needle disengagement sensing mechanism 10 is depicted. The mechanism 10 generally includes a fistula needle 12, an electronic sensing system 14, 14', and an alarm control conduit 16.

The fistula needle 12 includes a body 18 having two opposed end regions 20, 22. One of the opposed end regions 20 is a fluid contacting end, and the other end region 22 is opposed to the fluid contacting end region 20. It is to be understood that the needle 12 is adapted to carry fluid (a non-limitative example of which is a bodily fluid (e.g. blood) of a patient P). In an embodiment when the needle 12 is in an engaged position, the fluid contacting end region 20 receives fluid that flows through the other end region 22 to a fluid tubing set 24, which is fluidly connected to the other end region 22 of the needle 12. It is to be understood that the fluid tubing set 24 may be connected to a medical device, such as, for example a hemodialysis machine.

Non-limitative examples of the fistula needle 12 include monopolar needles, concentric needles, single-fiber needles, and/or the like. A monopolar needle may serve as an active electrode, and a separate skin surface electrode may be used as a reference electrode. Concentric needles are generally bipolar and have an insulated wire(s) in the center of the cannula. It is to be understood that the active electrode is the tip of the center wire, and the reference electrode is the outside cannula. Single-fiber needles are generally bipolar needles having enhanced sensitivity. A non-limitative example of a single-fiber needle has about a 0.5 mm to about a 0.6 mm stainless steel cannula with a 25 µm platinum wire in its hollow shaft, where a portion of the platinum wire is exposed.

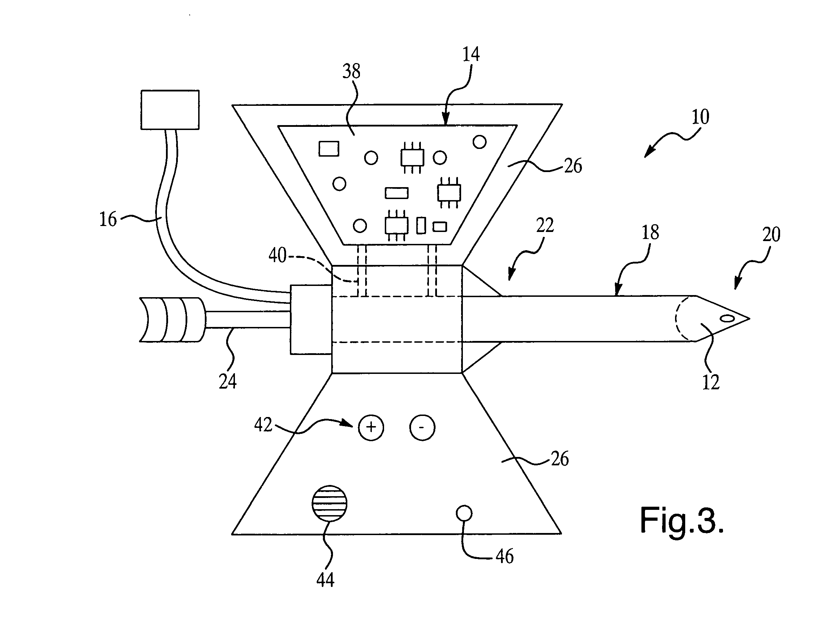

In the embodiment depicted in Fig. 2, the electronic sensing system 14 is housed near the end region 22 opposed to the fluid contacting end region 20 of the needle 12. In an alternate embodiment, the electronic sensing system 14' is integrated into the body 18 of the fistula needle 12. In still a further embodiment, the electronic sensing system 14 is integrated in securing tabs 26 that are attached to the body 18 of the needle 12 (see Fig. 3). Generally the securing tabs 26 may be used to secure the needle 12 to the vascular access point of the patient P.

The electronic sensing system 14, 14' is capable of detecting partial or full disengagement of the fistula needle 12 from a position engaged with the fluid. The fluid or patient engaged position occurs when the needle 12 is fully inserted at a vascular access point V on the patient P. The fluid or patient disengaged position occurs when the needle 12 become partially or fully disengaged form the vascular access point V.

In an embodiment, the electronic sensing system 14, 14' measures the complex impedance of the needle 12, both when the needle 12 is in, and when it 12 is at least partially out of the engaged position. Examples of complex impedances that the electronic sensing system 14, 14' is capable of measuring include, but are not limited to inductance measurements, capacitance measurements, resistance measurements, magnetic measurements, and combinations thereof. Without being bound to any theory, it is believed that the results of the complex impedance measurements of the needle 12 in the engaged position advantageously differ from the results of the measurements of the needle 12 when disengaged. As such, the detection of disengagement results from a comparative analysis of the complex impedance measurements of the needle 12. It is to be understood that the electronic sensing system 14, 14' is capable of comparing the various measurements and distinguishing between the various measurements.

In a non-limitative example, resistance impedance may be measured via a conductive strip or contact on the needle body 18 and a reference contact connected to a ground potential. In another non-limitative example, resistance impedance may be measured via a metallic needle body 18 and a return contact in relatively close proximity to the body 18 of the needle 12.

The electronic sensing system 14, 14' may include any suitable electronic circuitry, sensors, and/or combination thereof. Non-limitative examples of suitable sensors include conductive sensors, capacitive sensors, inductive sensors, resistance sensors, magnetic sensors, optical sensors and/or combinations thereof. It is to be understood that the configuration of the sensors may be passive or active to improve the signal-to-noise ratio characteristics of the physiological measurement. With a passive configuration, the signal is measured without the addition of other signals, and in an active configuration, additional signals are provided to the sensor during measurements to improve sensor sensitivity.

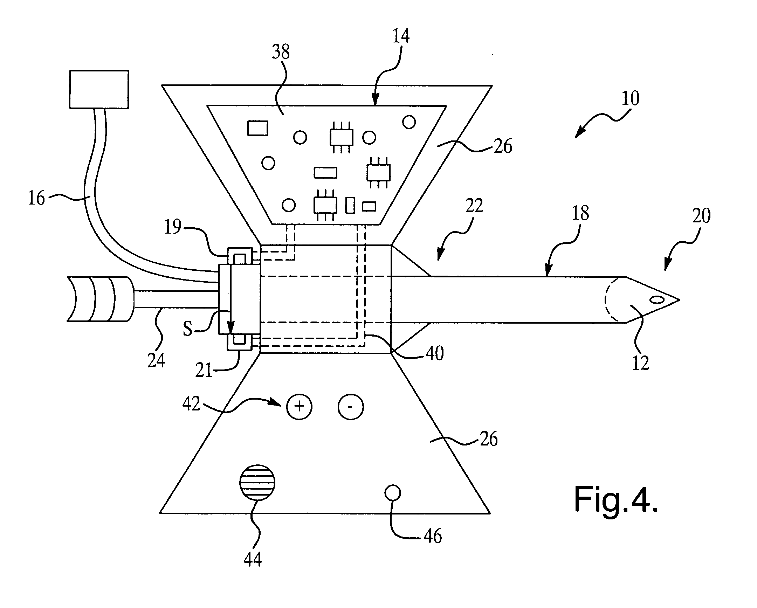

Fig. 2 also depicts an embodiment where the electronic sensing system 14 includes optical sensors or an optical spectroscopic sensing system (shown as optical transmitter 19, optical transducer/receiver 21, and reflector 23). The optical transmitter 19 is adapted to transmit an optical signal S across an area of the mechanism 10 (e.g. across the width of the needle 12) that is adapted to carry the fluid therein. It is to be understood that the optical signal S is transmitted such that fluid may flow through the signal S. The optical transducer 21 is adapted to detect the optical signal S transmitted from the optical transmitter 19. The signal S may be transmitted directly from the transmitter 19 to the transducer 21 (see Fig. 4), or may be reflected back to the transducer 21 via a reflector 23 (as shown in Fig. 2). In the embodiment shown in Fig. 2, the optical transmitter 19 and optical transducer 21 are adjacent each other and are affixed opposed to the reflector 23.

The optical signal S emitted by the optical transmitter 19 may be infrared radiation, ultraviolet radiation, visible radiation, and/or combinations thereof. It is to be understood that the peak wavelength of the optical transmitter 19 and the sensitivity of the optical transducer 21 may be optimized to maximize the signal-to-noise ratio.

When the needle 12 is in the engaged position, fluid flows through the area across which the optical signal S is transmitted. In this embodiment, a relatively large optical attenuation may be measured. When the needle 12 is at least partially disengaged, the amount of fluid flowing through the area where the optical signal S is transmitted across decreases, resulting in a smaller optical attenuation. As such, the detection of disengagement results from a comparative analysis of the optical spectroscopic measurements (i.e. optical transmission characteristics) taken through the area when the needle 12 is in and at least partially out of the engaged position.

It is to be understood that the electronic sensing system 14, 14' is capable of comparing the various measurements and distinguishing between the various measurements.

Yet further, it is to be understood that the electronic sensing system 14, 14' may be capable of conducting both complex impedance measurements and comparisons and optical spectroscopic measurements and comparisons. Incorporating both methods may advantageously provide varying forms of generating the alarm (described hereinbelow).

The electronic sensing system 14, 14' is operatively connected to the alarm control conduit 16. Fig. 2 depicts both a wired and a wireless alarm control conduit 16. It is to be understood that either configuration, or a combination of both configurations, may be suitable for the mechanism 10.

In the wired embodiment of the alarm control conduit 16, a cable 28 electrically connects the electronic sensing system 14, 14' to an electronic device 30 (non-limitative examples of which include pagers, computers, personal digital assistants (PDAs), cellular phones, and combinations thereof) and/or a hemodialysis machine 32. As depicted, the cable 28 electrically connects to a power outlet strip 34, where the hemodialysis machine 32 and/or the electronic device 30 may be plugged in.

In the wireless embodiment of the alarm control conduit 16, radio frequency or infrared means 36 electrically and operatively connects the electronic sensing system 14, 14' to the electronic device 30 and/or the hemodialysis machine 32. In a non-limitative example, a transmitter (not shown) is operatively connected to the electronic sensing system 14, 14', and a receiver (not shown) is operatively connected to the electronic device 30 and/or the hemodialysis machine 32. In this non-limitative example embodiment, the receiver may be positioned in, and electrically connected to, the power outlet strip 34.

Upon recognition of partial or full disengagement of the needle 12 from the engaged position, the electronic sensing system 14, 14' sends a signal to the alarm control conduit 16. In response, the alarm control conduit 16 is capable of generating an alarm. Non-limitative examples of the alarm include visual alarms, audio alarms, and/or combinations thereof.

In alternate embodiments, the alarm is capable of sending a signal to the electronic device 30 and/or automatically shutting down the hemodialysis machine 32.

In an embodiment where the alarm control conduit 16 is operatively connected (e.g. via a cable 28 or a wireless 36 connection) with the power strip 34, the alarm interrupts a main power supply to any device (e.g. hemodialysis machine 32) that is plugged into the modified power strip 34. As such, the device loses power and shuts down. It is to be understood that in this embodiment, the power strip 34 may be modified to include electronics capable of interrupting the power supply, thereby shutting down the machine 32, upon recognition of the alarm.

In an alternate embodiment not depicted in the figures, the alarm control conduit 16 is operatively connected to the electronic device 30, which is also operatively connected to the hemodialysis machine 32. In this embodiment, upon recognizing the alarm, the electronic device 30 signals the hemodialysis machine 32 to shut down. Still further, the electronic device 30 may also or alternately be programmed so that the patient P or other person may manually shut down the machine 32 via the electronic device 30 when the alarm is generated. It is to be understood that in this embodiment, the hemodialysis machine 32 may be modified to include electronics capable of shutting down the machine 32 upon recognition of the signal from the electronic device 30.

It is to be understood that the previous methods of automatic shutdown may be combined such that a safeguard mode is implemented if one of the shutdown mechanisms fails. The alarm conduit control 16 may also optionally be programmed to emit a visual and/or audible alarm.

Referring now to Fig. 3, a non-limitative embodiment of the electronic sensing system 14 is depicted. The system 14 is integrated in the securing tabs 26 that are attached to the needle 12. In this embodiment, the electronic sensing system 14 includes a printed circuit board assembly 38 that is electrically connected to the needle 12 via electrical lead(s) 40.

Fig. 3 also depicts optional batteries 42, an audible alarm annunciator 44, and a visible alarm annunciator 46. It is to be understood that the audible and visible alarm annunciators 44, 46 are components of the alarm control conduit 16, but may be integrated with the securing tabs 26.

Referring now to Fig. 4, another non-limitative embodiment of the electronic sensing system 14 is depicted. The electronic sensing system 14 includes an optical sensor (shown as optical transmitter 19 and optical transducer 21). A portion of the electronic sensing system 14 is integrated in the securing tabs 26 that are attached to the needle 12. In this embodiment, the electronic sensing system 14 includes a printed circuit board assembly 38 that is electrically connected to the optical sensor 19, 21 via electrical lead(s) 40. In this non-limitative example, the optical transmitter 19 and the optical transducer 21 are affixed at opposed regions near the end 22 of the needle 12, such that the optical signal S is transmitted across an area of the mechanism 10.

Fig. 4 also depicts the batteries 42, the audible alarm annunciator 44, and the visible alarm annunciator 46, all of which are described in reference to Fig. 3.

Embodiment(s) of the mechanism 10 and method include, but are not limited to the following advantages. Detection at a desired level of sensitivity of partial or full disengagement of the needle 12 may be accomplished using the mechanism 10. Without being bound to any theory, this may be due, at least in part, to the direct impedance measurements of the needle 12 and/or the optical measurements. Further, the automatic shutdown of the hemodialysis machine 32 enables the treatment of patient P to be discontinued without the assistance of another person (e.g. a caregiver). Still further, embodiment(s) of the mechanism 10 incorporate measuring complex impedance of the needle and/or optical signals of an area of the mechanism 10 adapted to carry fluid via an electronic sensing system 14 that may be passive or active.

While several embodiments have been described in detail, it will be apparent to those skilled in the art that the disclosed embodiments may be modified. Therefore, the foregoing description is to be considered exemplary rather than limiting.

## Claims

1. A needle disengagement sensing mechanism, comprising:
a fistula needle adapted to carry a fluid;
an electronic sensing system operatively connected to the fistula needle, the electronic sensing system capable of detecting at least partial disengagement of the fistula needle from a position engaged with the fluid, the detecting being a result of at least one of i) a comparative analysis of complex impedance measurements of the needle when in the fluid engaged position and when at least partially out of the fluid engaged position and ii) a comparative analysis of optical spectroscopic measurements taken through an area of the mechanism adapted to carry the fluid, the measurements being taken when the needle is in the fluid engaged position and when the needle is at least partially out of the fluid engaged position; and
an alarm control conduit operatively connecting the electronic sensing system to at least one of a hemodialysis machine and an electronic device, wherein the alarm control conduit generates an alarm upon detecting at least partial disengagement of the fistula needle.

2. The needle disengagement sensing mechanism as defined in claim 1 wherein the alarm control conduit operatively connects the electronic sensing system to the hemodialysis machine, and wherein the hemodialysis machine is adapted to shut down upon the alarm control conduit generating the alarm.

3. The needle disengagement sensing mechanism as defined in claim 2 wherein the alarm control conduit and the hemodialysis machine are electrically connected via a power outlet strip, and wherein the alarm interrupts a main power supply to the hemodialysis machine, thereby shutting down the machine.

4. The needle disengagement sensing mechanism as defined in claim 3 wherein the operative connection between the alarm control conduit and the hemodialysis machine is wireless.

5. The needle disengagement sensing mechanism as defined in claim 4 wherein the wireless operative connection is accomplished with a transmitter and a receiver.

6. The needle disengagement sensing mechanism as defined in claim 1 wherein the fistula needle has a fluid contacting end and an end region opposed to the fluid contacting end, and wherein the electronic sensing system is housed at the end region opposed to the fluid contacting end.

7. The needle disengagement sensing mechanism as defined in claim 6 wherein the electronic sensing system is integrated into a body of the fistula needle adjacent the end region opposed to the fluid contacting end.

8. The needle disengagement sensing mechanism as defined in claim 1 wherein a portion of the electronic sensing system is in contact with the needle.

9. The needle disengagement sensing mechanism as defined in claim 1 wherein the electronic sensing system includes at least one of conductive sensors, capacitive sensors, inductive sensors, resistance sensors, magnetic sensors, optical sensors, and combinations thereof.

10. The needle disengagement sensing mechanism as defined in claim 9 wherein the optical sensor includes a transmitter and a receiver.

11. The needle disengagement sensing mechanism as defined in claim 1 wherein the optical spectroscopic measurements include optical signal transmission characteristics.

12. The needle disengagement sensing mechanism as defined in claim 1 wherein complex impedance measurements include at least one of inductance measurements, capacitance measurements, resistance measurements, magnetic measurements, and combinations thereof.

13. The needle disengagement sensing mechanism as defined in claim 1 wherein the fluid is a body fluid of a patient.

14. The needle disengagement sensing mechanism as defined in claim 1 wherein the electronic device is at least one of pagers, computers, personal digital assistants (PDAs), cellular phones, and combinations thereof.

15. The needle disengagement sensing mechanism as defined in claim 14 wherein the operative connection between the alarm control conduit and the electronic device is wireless.

16. The needle disengagement sensing mechanism as defined in claim 14 wherein the alarm control conduit operatively connects the electronic sensing system to the electronic device, wherein the electronic device is operatively connected to the hemodialysis machine, and wherein the hemodialysis machine is adapted to be shut down from the electronic device.

17. The needle disengagement sensing mechanism as defined in claim 1 wherein the electronic sensing system is capable of detecting full disengagement of the fistula needle from the position engaged with the fluid.

18. A method for detecting at least partial disengagement of a fistula needle from a patient, the method comprising:
inserting the fistula needle in the patient, the fistula needle being operatively connected to an electronic sensing system that is operatively connected to an alarm control conduit;
measuring, via the electronic sensing system, at least one of complex impedance of the needle and optical signals through an area adapted to carry fluid, the electronic sensing system capable of at least one of i) distinguishing between complex impedance when the needle is in a patient engaged position and complex impedance when the needle is in an at least partially patient disengaged position, and ii) distinguishing between an optical signal when the needle is in the patient engaged position and an optical signal when the needle is in the at least partially disengaged position; and
generating an alarm upon recognition of the needle in the at least partially disengaged position.

19. The method as defined in claim 18 wherein the alarm control conduit operatively connects the electronic sensing system to a hemodialysis machine, and the method further comprises shutting down the hemodialysis machine when the alarm is generated.

20. The method as defined in claim 19 wherein shutting down the hemodialysis machine is accomplished automatically.

21. The method as defined in claim 20 wherein automatically shutting down the hemodialysis machine is accomplished by the alarm interrupting a main power supply to the hemodialysis machine, thereby shutting down the machine.

22. The method as defined in claim 20 wherein an electronic device is operatively connected to the electronic sensing system and the hemodialysis machine, wherein the electronic device is alerted when the alarm is generated, and wherein automatically shutting down the hemodialysis machine is accomplished via a signal transmitted from the electronic device to the hemodialysis machine.

23. The method as defined in claim 18 wherein the complex impedance measurements include at least one of inductance measurements, capacitance measurements, resistance measurements, magnetic measurements, and combinations thereof.

24. The method as defined in claim 18 wherein the optical signals are generated through the area via a transmitter.

25. The method as defined in claim 24 wherein the optical signals are received by a receiver.

26. The method as defined in claim 25 wherein a reflector transmits the optical signals from the transmitter to the receiver.
